# EUROPEAN PATENT SPECIFICATION

(11) **EP 1 513 485 B1**
(45) Date of publication and mention of the grant of the patent: **04.05.2011**
(21) Application number: 03736954.3
(22) Date of filing: 10.06.2003
(51) Int. Cl.: A61Q 5/02, A61K 8/06

(54) **COMPOSITION CONTAINING A CATIONIC POLYMER WITH A HIGH CHARGE DENSITY AND A CONDITIONING AGENT**
ZUSAMMENSETZUNGEN AUS KATIONISCHEN POLYMEREN MIT HOHER LADUNGSDICHTE UND KONDITIONIERUNGSMITTEL
COMPOSITION CONTENANT UN POLYMERE CATIONIQUE PRESENTANT UNE DENSITE DE CHARGE ELEVEE ET UN AGENT DE TONIFICATION CAPILLAIRE

(30) Priority: 18.06.2002 US 389634 P
(43) Date of publication of application: 16.03.2005
(73) Proprietor: THE PROCTER & GAMBLE COMPANY, Cincinnati, OH 45202 (US)
(72) Inventor: GEARY, Nicholas, William, Cincinnati, OH 45242 (US); JOHNSON, Eric, Scott, Hamilton, OH 45011 (US)
(74) Representative: Hampton, Matthew John
(86) International application number: PCT/US2003/018157
(87) International publication number: WO 2003/105793

(56) References cited:
- WO-A-95/09599
- WO-A-03/032935
- US-A- 5 720 964
- US-A- 5 756 436
- US-A- 5 977 038

## Description

### FIELD

The present invention relates to a hair cleansing shampoo comprising cationic polymers with a high charge density and a conditioning agent. More specifically, it relates to a shampoo containing cationic polysaccharide polymers having a high charge density and a nano-emulsified conditioning agent.

### BACKGROUND

Human hair becomes soiled due to its contact with the surrounding atmosphere and, to a greater extent, from sebum secreted by the head. The build-up of the sebum causes the hair to have a dirty feel and an unattractive appearance. The soiling of the hair necessitates it being shampooed with frequent regularity.

Shampooing the hair cleans by removing excess soil and sebum. However, the shampooing process has disadvantages in that the hair is left in a wet, tangled and generally unmanageable state. Shampooing can also result in the hair becoming dry or "frizzy", and a loss of luster, due to removal of natural oils or other hair moisturizing materials. After shampooing, the hair can also suffer from a loss of "softness" perceived by the user upon drying. The hair can also suffer from increased levels of static upon drying after shampooing. This can interfere with combing and can result in fly-away hair. A variety of approaches have been developed to alleviate the after-shampoo problems. These range from the inclusion of hair conditioning aids in shampoos to post-shampoo application of hair conditioners, i.e., hair rinses. Hair rinses are generally liquid in nature and must be applied in a separate step following the shampooing, left on the hair for a length of time, and rinsed with fresh water. This, of course, is time consuming and is not as convenient as shampoos containing both cleaning and hair conditioning ingredients. Therefore, it is desirable to have a shampoo capable of depositing conditioning aids.

Depositing materials such as conditioning aids from a shampoo composition can be difficult Deposition must be balanced against other factors such as cleansing properties of the shampoo, "feel" of the shampoo during use, and hair feel post-shampoo. Current polymers used as deposition aids are not always effective at depositing materials while maintaining the balance described above.

Silicone fluids suitable for use in the personal cleansing compositions have been disclosed in several patents dating back to the early 1970's, for example U.S. PaL No. 2,826,551, U.S. Pat. No. 3,964,500, U.S. Pat. No. 4,364,837, U.S. Pat. No. 4,788,006, GB Pat. No. 849,433, and *Silicon Compounds,* Petrarch Systems, Inc. (1984), all of which teach the use of silicone fluids.

The use of cationic polymers within personal cleansing applications has also been disclosed. Of particular interest to this application is the use of cationic polymers to aid in the deposition of conditioning actives such as silicones. U.S. patent 4,364,837 (Pader 1982) teaches the use of both cationic polymers and silicones in a shampoo composition. In the patent, a preferred hair grooming agent system is a mixture of silicone and a cationic cellulose, and the most preferred hair grooming agent is a mixture of polydimethylsiloxane and polymer JR. This patent further teaches the use of cationic celluloses, cationic guars, such as Jaguar C-17 in combination with silicone.

U.S. Pat. No. 3,964,500 (Drakoff 1976) also teaches the combination of a cationic cellulose polymer, specifically polymer JR, and silicone, Further, Drakoff teaches the former (termed a hair bodying resin in the application) to enhance the deposition of the latter. Specifically, this reference teaches that the hair bodying resin precipitates upon dilution of the shampoo composition and application of the hair whereupon the resin coacervates with the siloxane and the coacervate deposits on the hair strands. This proposed mechanism for the formation of a coacervate precipitant that forms upon rinsing is now known to occur between the cationic polymer and surfactant. This phase separated complex is known in the art as a coacervate or floc, the former term being first used by H.G. Bungenberg De Jong in 1929 and literally means to "heap together". Without being bound by theory, it is believed the precipitated coacervate has a tendency to both deposit itself and aid in the deposition of suspended droplets or particles.

It remains, therefore, highly desirable to have a rinse-off composition, preferably a cleansing composition, capable of containing and effectively depositing and retaining conditioning aids on the surface treated therewith. It has now been discovered that select cationic polymers, when used in the cleansing compositions of the present invention, can surprisingly enhance the deposition and retention of conditioning benefit agents on the surfaces treated therewith.

### SUMMARY

The present invention is directed to a shampoo composition comprising:
a) from 4 to 50 weight percent of a detersive surfactant comprising anionic surfactant and betaines
b) at least 0.05 weight percent of a cationic polymer,
   i) wherein said cationic polymer has a molecular weight from 10,000 to 10,000,000; and
   ii) wherein said cationic polymer has a charge density from 1.4 meq/g to 7 meq/g;
c) at least 0.05 weight percent of a conditioning agent material having a volume average diameter of less than 1.0 microns; and
d) at least 20.0 weight percent of an aqueous carrier.

The present invention is further directed to a cosmetic method of using the shampoo composition.

These and other features, aspects, and advantages of the present invention will become evident to those skilled in the art from a reading of the present disclosure.

### DETAILED DESCRIPTION

While the specification concludes with claims that particularly point out and distinctly claim the invention, it is believed the present invention will be better understood from the following description.

One embodiment of the present invention concerns the surprising discovery that compositions combining certain high charge density cationic polymers in combination with surfactants, forms, upon rinsing, a preferred microscopically-phase separate coacervate suspended in an aqueous surfactant phase. In use, the dispersed, coacervate phase provides improved conditioning agent deposition versus that previously known in the art. A further embodiment of the present invention concerns the surprising discovery that particular charge density cationic polymers yield yet higher levels of conditioning agent deposition. The following table exemplifies several of the highly preferred polymers and their charge density and also contains the same information for "non preferred" cationic polymers previously disclosed. A further embodiment of the present invention concerns the surprising discovery that compositions combining the certain high charge density cationic polymers are particularly effective at aiding in the deposition of the conditioning agent when the condition agent is below 1 micron, more preferably below 0.3 microns and even more preferably below 0.1 microns. We herein define nanoemulsions as conditioning agent materials with a volume average diameter of less than 1 micron.

As noted, the use of cationic polymers to aid in the deposition of silicones - without regard to particle size - was taught by Pader (1982) and Drakoff (1976) - the latter specifically teaching Polymer JR400 and the former teaching Polymer JR and cationic Guars. The first reference to very small silicone particles and or microemulsions occurred in the 1980's when this class of silicones was commercialised by the silicone industry. The Toray silicone company in European patent 0268982 (Priority date of Nov 1986, JP 274799/86) teach the use of dimethlpolysiloxane microemulsions with a preferred particle size of less than 0.15 microns in combination with a cationic polymer, specifically Merquat 550, in a shampoo context. Gee at al. teach the combination of surfactants, cationic polymers and an aqueous siloxane microemulsion in a hair fixative preparation Gee et al., specifically teach the use of Jaguar 400.

Birtwistle in European pat. No. 0529883A1 also teach the use of silicones with a particles size less then 0.15 microns with cationic deposition polymers, specifically teaching the use of Jaguar C-13S, Jaguar C-15, Jaguar C-17, Jaguar C-162, JR400 and JR30M. These cationic polymers all have low charge densities, while the preferred cationic polymers of the present invention have high charge densities.

| | Charge Density Meq/gm | |
|---|---|---|
| Jaguar C17 (1) | 0.9 | Taught in US 4.364,837 |
| JR400 (2) | 1.25 | Taught in US 4,364,837 |
| Jaguar C-13S (3) | 0.5 | Taught in EP 0529883 |
| Jaguar C 162 (4) | 0.3 | Taught in EP 0529883 |
| Polyquaternium 10 - JR30M (5) | 1.25 | Taught in EP 0529883 |
| Guar Hydroxypropyl trimonium chloride (6) | 2.40 | Highly Preferred |
| Guar Hydroxypropyl trimonium chloride (7) | 2.10 | Preferred |
| Guar Hydroxypropyl trimonium chloride (8) | 2.10 | Preferred |
| Guar Hydroxypropyl trimonium chloride (9) | 1.57 | Preferred |
| Polyquaternium 10 (10) | 1.97 | Highly Preferred |
| Polyquaternium 10 (11) | 2.38 | Highly preferred |
| Polyquaternium 10 (12) | 2.39 | Highly Preferred |
| Polyquaternium 10 (13) | 1.79 | Preferred |
| Polyquaternium 10 (14) | 2.74 | Preferred |

| | | |
|---|---|---|
| 1. Guar Hydroxypropyl trimonium chloride Jaguar C17 available from Rhodia and having a charge density of 0.9 meq/gm and a molecular weight of 2,200,000 2. Polyquaternium 10 - Polymer JR400 available from Amerchol/Dow Chemical and having a charge density of 1.25 meq/gm and a molecular weight of 400,000 3. Guar Hydroxypropyl trimonium chloride Polymer Jaguar C-13S available from Rhodia and having a charge density pf 0.5 meq/gm and a molecular weight of 2,200,000. 4. Guar Hydroxypropyl trimonium chloride Polymer Jaguar C162 available from Rhodia and having a charge density of 0.3 meq/gm 5. Polyquaternium 10 - Polymer JR30M available from Amerchol/Dow Chemical and having a charge density of 1.25 meq/gm and a molecular weight of 1,800,000 6. Guar having a molecular weight of about 600,000, and having a charge density of about 2.40 meq/g, available from Aqualon. 7. Guar having a molecular weight of about 400,000, and having a charge density of about 2.10 meq/g, available from Aqualon. 8. Guar having a molecular weight of about 1,100,000, and having a charge density of about 2.10 meq/g, available from Aqualon. 9. Guar having a molecular weight of about 400,000, and having a charge density of about 1.57 meq/g, available from Aqualon. 10. Polymer KG30M available from Amerchol/Dow with a charge density of 1.97 meq/gm and a molecular weight of 2,000,000 11. Cationic cellulose polymer available from Amerchol/Dow with a charge density of 2.38 meq/gm and a molecular weight of 2,000,000 12. Cationic cellulose polymer available from Amerchol/Dow with a charge density of 2.39 meq/gm and a molecular weight of 400,000 13. Cationic cellulose polymer available from Amerchol/Dow with a charge density of 1.79 meq/gm and a molecular weight of 2,000,000 14. Cationic cellulose polymer available from Amerchol/Dow with a charge density of 2.74 meq/gm and a molecular weight of 450,000 | | |

The shampoo compositions of the present invention include detersive surfactant, a cationic polymer, a nanoemulsified conditioning agent, and an aqueous carrier. Each of these essential components, as well as preferred or optional components, are described in detail hereinafter.

All percentages, parts and ratios are based upon the total weight of the compositions of the present invention, unless otherwise specified. All such weights as they pertain to listed ingredients are based on the active level and, therefore, do not include solvents or by-products that may be included in commercially available materials, unless otherwise specified.

All molecular weights as used herein are weight average molecular weights expressed as grams/mole, unless otherwise specified.

The term "nanoemulsion", as used herein, is an emulsion made with a conditioning agent material, with a volume average diameter of less than 1 micron. The emulsion may or may not be chemically stable. It may be formed mechanically or via the use of appropriate surfactants.

The term "charge density", as used herein, refers to the ratio of the number of positive charges on a polymer to the molecular weight of said polymer.

Herein, "comprising" means that other steps and other ingredients which do not affect the end result can be added. This term encompasses the terms "consisting of" and "consisting essentially of". The compositions and methods/processes of the present invention can comprise, consist of, and consist essentially of the essential elements and limitations of the invention described herein, as well as any of the additional or optional ingredients, components, steps, or limitations described herein.

The term "polymer" as used herein shall include materials whether made by polymerization of one type of monomer or made by two (*i.e.*, copolymers) or more types of monomers.

The term "suitable for application to human hair" as used herein, means that the compositions or components thereof so described are suitable for use in contact with human hair and the scalp and skin without undue toxicity, incompatibility, instability, allergic response, and the like.

The term "water soluble" as used herein, means that the polymer is soluble in water in the present composition. In general, the polymer should be soluble at 25° C at a concentration of 0.1% by weight of the water solvent, preferably at 1%, more preferably at 5%, even more preferably at 15%.

### A. Detersive Surfactant

The personal cleansing composition useful in the present invention includes a surfactant selected from a group consisting of anionic, cationic, nonionic, amphoteric, and zwitterionic surfactants and mixtures thereof. The surfactant system of the present invention is preferably present in the personal cleansing compositions at a level of from 4% to 50%, more preferable from 4% to 40%, still more preferably from 4% to 30%, even more preferably from 5% to 20% and even more preferably from 6% to 16%. It should be recognized, however, that the concentration of the surfactant system may vary with the cleaning or lather performance desired, the surfactants incorporated into the surfactant system, the desired product concentration, the presence of other components in the composition, and other factors well known in the art.

Suitable anionic detersive surfactant components for use in the shampoo composition herein include those which are known for use in hair care or other personal care cleansing compositions. The concentration of the anionic surfactant component in the shampoo composition should be sufficient to provide the desired cleaning and lather performance, and generally range from 5% to 50%, preferably from 8% to 30%, more preferably from 10% to 25%, even more preferably from 12% to 18%, by weight of the composition.

Preferred anionic surfactants suitable for use in the shampoo compositions are the alkyl and alkyl ether sulfates. These materials have the respective formulae ROSO₃M and RO(C₂H₄O)xS0₃M, wherein R is alkyl or alkenyl of from about 8 to about 18 carbon atoms, x is an integer having a value of from 1 to 10, and M is a cation such as ammonium, alkanolamines, such as triethanolamine, monovalent metals, such as sodium and potassium, and polyvalent metal cations, such as magnesium, and calcium.

Preferably, R has from 8 to 18 carbon atoms, more preferably from 10 to 16 carbon atoms, even more preferably from 12 to 14 carbon atoms, in both the alkyl and alkyl ether sulfates. The alkyl ether sulfates are typically made as condensation products of ethylene oxide and monohydric alcohols having from 8 to 24 carbon atoms. The alcohols can be synthetic or they can be derived from fats, e.g., coconut oil, palm kernel oil, tallow. Lauryl alcohol and straight chain alcohols derived from coconut oil or palm kernel oil are preferred. Such alcohols are reacted with between 0 and 10, preferably from 2 to 5, more preferably about 3, molar proportions of ethylene oxide, and the resulting mixture of molecular species having, for example, an average of 3 moles of ethylene oxide per mole of alcohol, is sulfated and neutralized.

Other suitable anionic detersive surfactants are the water-soluble salts of organic, sulfuric acid reaction products conforming to the formula [R¹-SO₃-M] where R¹ is a straight or branched chain, saturated, aliphatic hydrocarbon radical having from 8 to 24, preferably 10 to 18, carbon atoms; and M is a cation described hereinbefore.

Still other suitable anionic detersive surfactants are the reaction products of fatty acids esterified with isethionic acid and neutralized with sodium hydroxide where, for example, the fatty acids are derived from coconut oil or palm kernel oil; sodium or potassium salts of fatty acid amides of methyl tauride in which the fatty acids, for example, are derived from coconut oil or palm kernel oil. Other similar anionic surfactants are described in U.S. Patent 2,486,921; U.S. Patent 2,486,922; and U.S. Patent 2,396,278, U.S. Patent 3,332,880 and U.S. Patent 5,756,436 (Royce et al.).

Preferred anionic detersive surfactants for use in the shampoo compositions include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, and combinations thereof.

Suitable amphoteric or zwitterionic detersive surfactants for use in the shampoo composition herein include those which are known for use in hair care or other personal care cleansing composition, and which contain a group that is anionic at the pH of the shampoo composition. Concentration of such amphoteric detersive surfactants preferably ranges from 0.5 % to 20%, preferably from 1% to 10%, by weight of the composition. Non limiting examples of suitable zwitterionic or amphoteric surfactants are described in U.S. Patents 5,104,646 (Bolich Jr. et al.), U.S. Patent 5,106,609 (Bolich Jr. et al.).

Amphoteric detersive surfactants suitable for use in the shampoo composition are well known in the art, and include those surfactants broadly described as derivatives of aliphatic secondary and tertiary amines in which the aliphatic radical can be straight or branched chain and wherein one of the aliphatic substituents contains from about 8 to about 18 carbon atoms and one contains an anionic water solubilizing group such as carboxy, sulfonate, sulfate, phosphate, or phosphonate.

Zwitterionic detersive surfactants suitable for use in the shampoo composition are well known in the art, and include those surfactants broadly described as derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one contains an anionic group such as carboxy, sulfonate, sulfate, phosphate or phosphonate. Zwitterionics such as betaines are preferred.

Cationic surfactants are also useful in compositions of the present invention and typically contain amino or quaternary ammonium hydrophilic moieties which are positively charged when dissolved in the aqueous composition of the present invention. Cationic surfactants among those useful herein are disclosed in the following documents : M.C. Publishing Co., McCutcheon's. Detergents & Emulsifiers, (North American edition 1989); Schwartz, et al., Surface Active Agents, Their Chemistry and Technology. New York: Interscience Publishers, 1949; U.S. Patent 3,155,591, Hilfer, issued November 3, 1964; U.S. Patent 3,929,678, Laughlin et al., issued December 30, 1975; U.S. Patent 3,959,461, Bailey et al., issued May 25, 1976; and U.S. Patent 4,387,090, Bolich, Jr., issued June 7, 1983. If included in the compositions of the present invention, the cationic surfactant must not interfere with the in-use performance and end-benefits of the personal cleansing composition.

The shampoo compositions of the present invention may further comprise additional surfactants for use in combination with the anionic detersive surfactant component described hereinbefore. Suitable optional surfactants include nonionic surfactants, cationic surfactants, and combinations thereof. Any such surfactant known in the art for use in hair or personal care products may be used, provided that the optional additional surfactant is also chemically and physically compatible with the essential components of the shampoo composition, or does not otherwise unduly impair product performance, aesthetics or stability. The concentration of the optional additional surfactants in the shampoo composition may vary with the cleansing or lather performance desired, the optional surfactant selected, the desired product concentration, the presence of other components in the composition, and other factors well known in the art.

Non limiting examples of other anionic, zwitterionic, amphoteric or optional additional surfactants suitable for use in the shampoo compositions are described in McCutcheon's. Emulsifiers and Detergents, 1989 Annual, published by M. C. Publishing Co., and U.S. Patent 3,929,678, U.S. Patent 2,658,072; U.S. Patent 2,438,091; U.S. Patent 2,528,378.

### B. Cationic Polymer

The composition of the present invention includes a cationic deposition polymer of sufficiently high cationic charge density to effectively enhance deposition of the nanoemulsion conditioning agent described herein. Suitable cationic polymers will have cationic charge densities of at least 1.4 meq/gm, preferably at least 1.6 meq/gm, more preferably at least 1.8 meq/gm, even more preferably at least 20 meq/gm, but also preferably less than 7 meq/gm, more preferably less than 5 meq/gm, still more preferably less than 4.0 meq/gm at the pH of intended use of the shampoo composition, which pH will generally range from pH 3 to pH 9, preferably between pH 4 and pH 8. The average molecular weight of such suitable cationic polymers will generally be between 10,000 and 10 million, preferably between 50,000 and 5 million, more preferably between 100,000 and 3 million.

The "cationic charge density" of a polymer, as that term is used herein, refers to the ratio of the number of positive charges on a polymer to the molecular weight of said polymer. The cationic charge density multiplied by the polymer molecular weight determines the number of positively charged sites on a given polymer chain. Charge Density is further defined as the number of mili-equivalents of charge (quaternary nitrogen) per gram (meq/g) of polymer. For cationic cellulose (described hereinafter) this is calculated from the %N value determined by Kjeldahl nitrogen determination. The cationic celluloses %N values were reported from Dow/ Amerchol and the meq/g was calculated by the inventors. Cationic guars (described hereinafter) measure degree of substitution, the number of hydroxyl groups substituted with cationic groups on a particular sugar moiety (maximum of three per sugar). Degree of substitution is used to account for the equivalents of N per sugar moiety, which then leads to the mili-equivalents of charge per gram of polymer. The cationic guar degree of substitution was determined by Aqualon/ Hercules and the meq/g was calculated by the inventors.

The concentration of the cationic polymer in the shampoo composition ranges from 0.05% to 3%, preferably from 0.075% to 2.0%, more preferably from 0.1% to 1.0%, by weight of the shampoo composition. The weight ratio of cationic polymer to a conditioning agent (described hereinafter) in the shampoo compositions is preferably from 4:1 to 1:40, more preferably from 2:1 to 1:20, still more preferably from 1:1 to 1:5.

The cationic polymers herein are either soluble in the shampoo composition or are soluble in a complex coacervate phase in the shampoo composition formed by the cationic polymer and the anionic detersive surfactant component described hereinbefore. Complex coacervates of the cationic polymer can also be formed with other charged materials in the shampoo composition.

Coacervate formation is dependent upon a variety of criteria such as molecular weight, component concentration, and ratio of interacting ionic components, ionic strength (including modification of ionic strength, for example, by addition of salts), charge density of the cationic and anionic components, pH, and temperature. Coacervate systems and the effect of these parameters have been described, for example, by J. Caelles, et al., "Anionic and Cationic Compounds in Mixed Systems", Cosmetics & Toiletries, Vol. 106, April 1991, pp 49-54, C. J. van Oss, "Coacervation, Complex-Coacervation and Flocculation", J. Dispersion Science and Technology, Vol. 9 (5,6), 1988-89, pp 561-573, and D. J. Burgess, "Practical Analysis of Complex Coacervate Systems", J. of Colloid and Interface Science, Vol. 140, No. 1, November 1990, pp 227-238.

It is believed to be particularly advantageous for the cationic polymer to be present in the shampoo composition in a coacervate phase, or to form a coacervate phase upon application or rinsing of the shampoo to or from the hair. Complex coacervates are believed to more readily deposit on the hair. Thus, in general, it is preferred that the cationic polymer exist in the shampoo composition as a coacervate phase or form a coacervate phase upon dilution.

Techniques for analysis of formation of complex coacervates are known in the art. For example, microscopic analyses of the shampoo compositions, at any chosen stage of dilution, can be utilized to identify whether a coacervate phase has formed. Such coacervate phase will be identifiable as an additional emulsified phase in the composition. The use of dyes can aid in distinguishing the coacervate phase from other insoluble phases dispersed in the shampoo composition.

In the compositions of the present invention, it is believed that the tendency for high charge density cationic polymers to form relatively large coacervates of sizes ranging from 20 microns to 500 microns which are capable of effectively binding or flocculating with the particle and enhancing delivery to hair contributes to the superior deposition efficiency. Additionally, coacervates which have a cohesive character as evidenced by large, structured flocs which retain a substantial amount of the particle component on dilution and resist deflocculation on exposure to shear enhance the deposition and retention of particles on hair.

A particular preferred class of cationic polymers are polysaccharide polymers. These can be further divided into polyanhydroglucose polymers and cationic guar derivatives.

### 1. Cationic Polyanhydroglucose Polymer

Cationic polymers useful in the present invention are polyanhydroglucose polymers, such as cationic cellulose derivatives and cationic starch derivatives. Suitable cationic polyanhydroglucose polymers include those which conform to the Formula I: 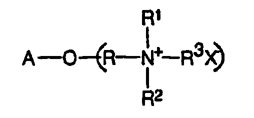 wherein A is an anhydroglucose residual group, such as a starch or cellulose anhydroglucose residual; R is an alkylene oxyalkylene, polyoxyalkylene, or hydroxyalkylene group, or combination thereof; R¹, R², and R³ independently are alkyl, aryl, alkylaryl, arylalkyl, alkoxyalkyl, or alkoxyaryl groups, each group containing up to about 18 carbon atoms, and the total number of carbon atoms for each cationic moiety (i.e., the sum of carbon atoms in R¹, R² and R³) preferably being about 20 or less; and X is an anionic counterion. Any anionic counterions can be use in association with the cationic polymers of the present invention so long as the polymers remain soluble in water, in the shampoo composition, or in a coacervate phase of the shampoo composition, and so long as the counterions are physically and chemically compatible with the essential components of the shampoo composition or do not otherwise unduly impair product performance, stability or aesthetics. Non limiting examples of such counterions include halides (e.g., chlorine, fluorine, bromine, iodine), sulfate and methylsulfate, and mixtures thereof. The degree of cationic substitution in these polysaccharide polymers is typically from 0.01-1 cationic groups per anhydroglucose unit.

Preferred cationic cellulose polymers salts of hydroxyethyl cellulose reacted with trimethyl ammonium substituted epoxide, referred to in the industry (CTFA) as Polyquaternium 10 and available from Amerchol Corp. (Edison, N.J., USA) as Polymer KG30M with a charge density of 1.9 and a molecular weight of - 1.25 million.

### 2. Cationic Guar Derivative

Another class of highly preferred cationic polymer is selected from a cationic guar derivative of sufficiently high cationic charge density to effectively enhance deposition of conditioning aids.

The cationic guars useful in the present invention must be selected and must be present at a level such that the cationic polymers are soluble in the shampoo composition, and which are preferably soluble in a complex coacervate phase in the shampoo composition, upon dilution. Such coacervate is described in detail hereinbefore. Physical properties of the cationic guars and suitable counterions are detailed hereinafter.

Guars are cationically substituted galactomannan (guar) gum derivatives. Guar gum for use in preparing these guar gum derivatives is typically obtained as a naturally occurring material from the seeds of the guar plant. The guar molecule itself is a straight chain mannan branched at regular intervals with single membered galactose units on alternative mannose units. The mannose units are linked to each other by means of β (1-4) glycosidic linkages. The galactose branching arises by way of an α (1-6) linkage. Cationic derivatives of the guar gums are obtained by reaction between the hydroxyl groups of the polygalactomannan and reactive quaternary ammonium compounds. The degree of substitution of the cationic groups onto the guar structure must be sufficient to provide the requisite cationic charge density described above.

Suitable quaternary ammonium compounds for use in forming the cationic guar polymers include those conforming to the general Formula (II): 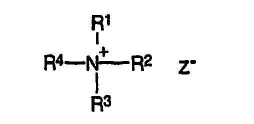 wherein where **R¹, R²** and **R³** are methyl or ethyl groups; **R⁴** is either an epoxyalkyl group of the general Formula (III): 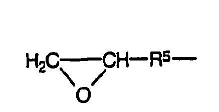 or **R⁴** is a halohydrin group of the general Formula (IV): 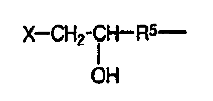 wherein **R⁵** is a C₁ to C₃ alkylene; **X** is chlorine or bromine, and **Z** is an anion such as Cl⁻, Br⁻, I⁻ or HSO4⁻.

Cationic guar polymers (cationic derivatives of guar gum) formed from the reagents described above are represented by the general Formula (V): 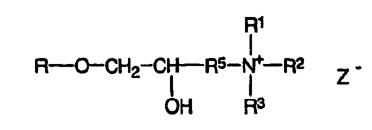 wherein R is guar gum. Preferably, the cationic guar polymer is guar hydroxypropyltrimethylammonium chloride, which can be more specifically represented by the general Formula (VI):

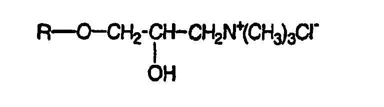

### Conditioning Agent

The conditioning agent consists of a nanoemulsion and includes any material which is used to give a particular conditioning benefit to hair and/or skin. In hair treatment compositions, suitable conditioning agents are those that deliver one or more benefits relating to shine, softness, combability, antistatic properties, wet-handling, damage, manageability, body, and greasiness. The conditioning agents useful in the personal cleansing compositions of the present invention typically comprise a water insoluble, water dispersible, non-volatile, liquid that forms emulsified, liquid particles or are solubilized by the surfactant micelles, in the anionic detersive surfactant component (described above). Suitable conditioning agents for use in the personal cleansing composition are those conditioning agents characterized generally as silicones (e.g. silicone oils, cationic silicones, silicone gums, high refractive silicones, and silicone resins), organic conditioning oils (e.g. hydrocarbon oils, polyolefins, and fatty esters) or combinations thereof, or those conditioning agents which otherwise form liquid, dispersed, particles in the aqueous surfactant matrix herein. Such conditioning agents should be physically and chemically compatible with the essential components of the composition, and should not otherwise unduly impair product stability, aesthetics or performance.

The concentration of the conditioning agent in the personal cleansing composition should be sufficient to provide the desired conditioning benefits, as will be apparent to one of ordinary skill in the art. Such concentration can vary with the conditioning agent, the conditioning performance desired, the average size of the conditioning agent particles, the type and concentration of other components, and other like factors.

The conditioning agent material typically has a volume average particle diameter of less than 1.0µm, more preferably of less than 0.3µm, still more preferably of less than 0.1µm. Even more preferably, the conditioning agent is an organopolysiloxane nanoemulsion.

### 1. Silicones

The conditioning agent of the personal cleansing compositions of the present invention preferably consists of a nanoemulsion of an insoluble silicone conditioning agent. The silicone conditioning agent material may comprise volatile silicone, con-volatile silicone, or combinations thereof. Preferred are non-volatile silicone conditioning agents. If volatile silicones are present, it will typically be incidental to their use as a solvent or carrier for commercially available forms of non-volatile silicone materials ingredients, such as silicone gums and resins. The silicone conditioning agent material may comprise a silicone fluid conditioning agent and may also comprise other ingredients, such as a silicone resin to improve silicone fluid deposition efficiency or enhance glossiness of the hair (especially when high refractive index (e.g. above about 1.46) silicone conditioning agents are used (e.g. highly phenylated silicones).

The concentration of the silicone conditioning agent typically ranges from 0.05% to 10%, by weight of the composition, preferably from 0.1% to 8%, more preferably from 0.1% to 5%, more preferably from 0.2% to 3%. Non-limiting examples of suitable silicone conditioning agents, and optional suspending agents for the silicone, are described in U.S. Reissue Pat. No. 34,584, U.S. Pat. No. 5,104,646, and U.S. PaL No. 5,106,609. The silicone conditioning agents for use in the personal cleansing compositions of the present invention preferably have a viscosity, as measured at 25°C, from 20 to 2,000,000 centistokes ("csk"), more preferably from 1,000 to 1,800,000 csk, even more preferably from 50,000 to 1,500,000 csk, more preferably from 100,000 to 1,500,000 csk.

The preferred nanoemulsion may be formed with any of the preferred conditioning agents dispersed in a suitable carrier (typically aqueous) with the aid of a surfactant.

Background material on silicones including sections discussing silicone fluids, gums, and resins, as well as manufacture of silicones, are found in Encyclopedia of Polymer Science and Engineering, vol. 15, 2d ed., pp 204-308, John Wiley & Sons, Inc. (1989).

### a. Silicone oils

Silicone fluids include silicone oils, which are flowable silicone materials having a viscosity, as measured at 25°C, less than 1,000,000 csk, preferably from 5 csk to 1,000,000 csk, more preferably from 10 csk to 100,000 csk. Suitable silicone oils for use in the personal cleansing compositions of the present invention include polyalkyl siloxanes, polyaryl siloxanes, polyalkylaryl siloxanes, polyether siloxane copolymers, and mixtures thereof. Other insoluble, non-volatile silicone fluids having hair conditioning properties may also be used.

Silicone oils include polyalkyl or polyaryl siloxanes which conform to the following Formula (VII): 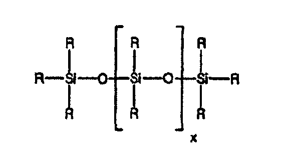 wherein R is aliphatic, preferably alkyl or alkenyl, or aryl, R can be substituted or unsubstituted, and x is an integer from 1 to about 8,000. Suitable unsubstituted R groups for use in the personal cleansing compositions of the present invention include, but are not limited to: alkoxy, aryloxy, alkaryl, arylalkyl, arylalkenyl, alkamino, and ether-substituted, hydroxyl-substituted, and halogen-substituted aliphatic and aryl groups. Suitable R groups also include cationic amines and quaternary ammonium groups.

Preferred alkyl and alkenyl substituents are C₁ to C₅ alkyls and alkenyls, more preferably from C₁ to C₄, more preferably from C₁ to C₂. The aliphatic portions of other alkyl-, alkenyl-, or alkynyl-containing groups (such as alkoxy, alkaryl, and alkamino) can be straight or branched chains, and are preferably from C₁ to C₅, more preferably from C₁ to C₄, even more preferably from C₁ to C₃, more preferably from C₁ to C₂. As discussed above, the R substituents can also contain amino functionalities (e.g. alkamino groups), which can be primary, secondary or tertiary amines or quaternary ammonium These include mono-, di- and tri- alkylamino and alkoxyamino groups, wherein the aliphatic portion chain length is preferably as described above.

### b. Cationic silicones

Cationic silicone fluids suitable for use in the personal cleansing compositions of the present invention include, but are not limited to, those which conform to the general formula (VIII):

(R₁)ₐG₃₋ₐ-Si-(-OSᵢG₂)ₙ-(-OSiG_{b}(R₁)_{2-b)m}-O-SiG₃₋ₐ(R₁)ₐ

wherein G is hydrogen, phenyl, hydroxy, or C₁-C₈ alkyl, preferably methyl; a is 0 or an integer having a value from 1 to 3, preferably 0; b is 0 or 1, preferably 1; n is a number from 0 to 1,999, preferably from 49 to 149; m is an integer from 1 to 2,000, preferably from 1 to 10; the sum of n and m is a number from 1 to 2,000, preferably from 50 to 500; R₁ is a monovalent radical conforming to the general formula CqH_{2q}L, wherein q is an integer having a value from 2 to 8 and L is selected from the following groups:
N(R₂)CH₂-CH₂-N(R₂)₂
-N(R₂)
-N(R₂)3A⁻
-N(R₂)CH₂-CH₂-NR₂H₂A⁻
wherein R₂ is hydrogen, phenyl, benzyl, or a saturated hydrocarbon radical, preferably an alkyl radical from about C₁ to about C₂₀, and A⁻ is a halide ion.

An especially preferred cationic silicone corresponding to formula (VIII) is the polymer known as "trimethylsilylamodimethicone", which is shown below in formula (IX):

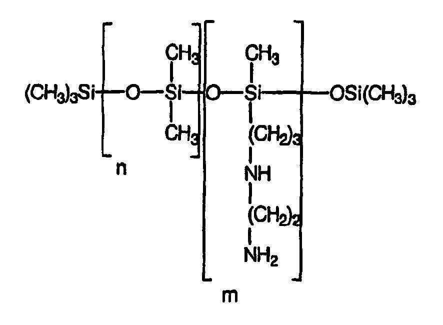

Other silicone cationic polymers which may be used in the personal cleansing compositions of the present invention are represented by the general formula (X): 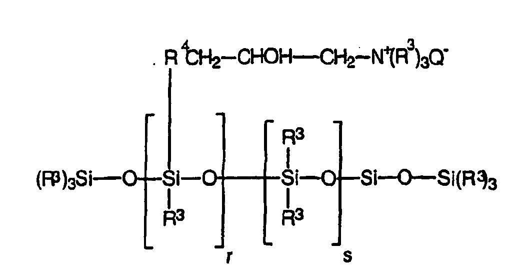 wherein R³ is a monovalent hydrocarbon radical from C₁ to C₁₈, preferably an alkyl or alkenyl radical, such as methyl; R₄ is a hydrocarbon radical, preferably a C₁ to C₁₈ alkylene radical or a C₁₀ to C₁₈ alkyleneoxy radical, more preferably a C₁ to C₈ alkyleneoxy radical; Q⁻ is a halide ion, preferably chloride; r is an average statistical value from 2 to 20, preferably from 2 to 8; s is an average statistical value from 20 to 200, preferably from 20 to 50. A preferred polymer of this class is known as UCARE SILICONE ALE 56™, available from Union Carbide.

### c. Silicone gums

Other silicone fluids suitable for use in the personal cleansing compositions of the present invention are the insoluble silicone gums. These gums are polyorganosiloxane materials having a viscosity, as measured at 25°C, of greater than or equal to 1,000,000 csk. Silicone gums are described in U.S. Pat. No. 4,152,416; Noll and Walter, Chemistry and Technology of Silicones, New York: Academic Press (1968); and in General Electric Silicone Rubber Product Data Sheets SE 30, SE 33, SE 54 and SE 76. The silicone gums will typically have a weight average molecular weight in excess of about 200,000, preferably from 200,000 to 1,000,000. Specific non-limiting examples of silicone gums for use in the personal cleansing compositions of the present invention include polydimethylsiloxane, (polydimethylsiloxane) (methylvinylsiloxane) copolymer, poly(dimethylsiloxane) (diphenyl siloxane)(methylvinylsiloxane) copolymer and mixtures thereof.

### d. High refractive index silicones

Other non-volatile, insoluble silicone fluid conditioning agents that are suitable for use in the personal cleansing compositions of the present invention are those known as "high refractive index silicones," having a refractive index of at least about 1.46, preferably at least about 1.48, more preferably at least about 1.52, more preferably at least about 1.55. The refractive index of the polysiloxane fluid will generally be less than about 1.70, typically less than about 1.60. In this context, polysiloxane "fluid" includes oils as well as gums.

The high refractive index polysiloxane fluid includes those represented by general Formula (VII) above, as well as cyclic polysiloxanes such as those represented by Formula (XI) below: 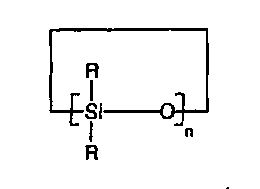 wherein R is as defined above, and n is a number from 3 to 7, preferably from 3 to 5.

The high refractive index polysiloxane fluids contain an amount of aryl-containing R substituents sufficient to increase the refractive index to the desired level, which is described above. Additionally, R and n must be selected so that the material is non-volatile.

Aryl-containing substituents include those which contain alicyclic and heterocyclic five and six member aryl rings and those which contain fused five or six member rings. The aryl rings themselves can be substituted or unsubstituted.

Generally, the high refractive index polysiloxane fluids will have a degree of aryl-containing substituents of at least about 15%, preferably at least about 20%, more preferably at least about 25%, even more preferably at least about 35%, more preferably at least about 50%. Typically, the degree of aryl substitution will be less than about 90%, more generally less than about 85%, preferably from 55% to 80%.

Preferred high refractive index polysiloxane fluids have a combination of phenyl or phenyl derivative substituents (more preferably phenyl), with alkyl substituents, preferably C₁-C₄ alkyl (more preferably methyl), hydroxy, or C₁-C₄ alkylamino (especially -R¹NHR²NH2 wherein each R¹ and R² independently is a C₁-C₃ alkyl, alkenyl, and/or alkoxy).

When high refractive index silicones are used in the personal cleansing compositions of the present invention, they are preferably used in solution with a spreading agent, such as a silicone resin or a surfactant, to reduce the surface tension by a sufficient amount to enhance spreading and thereby enhance the glossiness (subsequent to drying) of hair treated with the compositions.

Silicone fluids suitable for use in the personal cleansing compositions of the present invention are disclosed in U.S. Pat. No. 2,826,551, U.S. Pat. No. 3,964,500, U.S. Pat. No. 4,364,837, British Pat. No. 849,433, and Silicon Compounds, Petrarch Systems, Inc. (1984).

### e. Silicone resins

Silicone resins may be included in the silicone conditioning agent of the personal cleansing compositions of the present invention. These resins are highly cross-linked polymeric siloxane systems. The cross-linking is introduced through the incorporation of trifunctional and tetrafunctional silanes with monofunctional or difunctional, or both, silanes during manufacture of the silicone resin.

Silicone materials and silicone resins in particular, can conveniently be identified according to a shorthand nomenclature system known to those of ordinary skill in the art as "MDTQ" nomenclature. Under this system, the silicone is described according to presence of various siloxane monomer units which make up the silicone. Briefly, the symbol M denotes the monofunctional unit (CH3)₃SiO_{0.5}; D denotes the difunctional unit (CH₃)₂SiO; T denotes the trifunctional unit (CH₃)SiO_{1.5}; and Q denotes the quadra- or tetra-functional unit SiO₂. Primes of the unit symbols (e.g. M', D', T', and Q') denote substituents other than methyl, and must be specifically defined for each occurrence.

Preferred silicone resins for use in the personal cleansing compositions of the present invention include, but are not limited to MQ, MT, MTQ, MDT and MDTQ resins. Methyl is a preferred silicone substituent. Especially preferred silicone resins are MQ resins, wherein the M:Q ratio is from 0.5:1.0 to 1.5:1.0 and the average molecular weight of the silicone resin is from 1000 to 10,000.

The weight ratio of the non-volatile silicone fluid, having refractive index below 1.46, to the silicone resin component, when used, is preferably from 4:1 to 400:1, more preferably from 9:1 to 200: 1, more preferably from 19:1 to 100:1, particularly when the silicone fluid component is a polydimethylsiloxane fluid or a mixture of polydimethylsiloxane fluid and polydimethylsiloxane gum as described above. Insofar as the silicone resin forms a part of the same phase in the compositions hereof as the silicone fluid, i.e. the conditioning active, the sum of the fluid and resin should be included in determining the level of silicone conditioning agent in the composition.

### f. Aminosilicone

Herein "aminosilicone" means any amine functionalized silicone; i.e., a silicone containing at least one primary amine, secondary amine, tertiary amine, or a quaternary ammonium group. Preferred aminosilicones will typically have less than about 0.5% nitrogen by weight of the aminosilicone, more preferably less than about 0.2%, more preferably still, less than about 0.15%. Higher levels of nitrogen (amine functional groups) in the aminosilicone tend to result in very low deposition of the aminosilicone to the hair; and consequently, minimal to no conditioning benefit from the aminosilicone component.

In a preferred embodiment, the aminosilicone has a viscosity of from 1,000cs to 50,000cs, more preferably 2,000cs to 30,000cs, more preferably from 4,000cs to 20,000cs. The viscosity of the aminosilicone is more critical in embodiments which contain the aminosilicone as the only silicone component. However, in embodiments that contain aminosilicone in combination with a NAFS, the viscosity of the aminosilicone component becomes less critical when the aminosilicone makes up the minority of the total silicone in such a multi-silicone containing embodiment.

Example preferred aminosilicones for use in embodiments of the subject invention include but are not limited to, those which conform to the general formula (XII):

(R₁)ₐG₃₋ₐ-Si-(-OSiG₂)ₙ-(-OSiG_{b}(R₁)_{2-b)m}-O-SiG₃₋ₐ(R₁)ₐ

wherein G is hydrogen, phenyl, hydroxy, or C₁-C₈ alkyl, preferably methyl; a is 0 or an integer having a value from 1 to 3, preferably 1; b is 0, 1 or 2, preferably 1; n is a number from 0 to 1,999, preferably from 49 to 500; m is an integer from 1 to 2,000, preferably from 1 to 10; the sum of n and m is a number from 1 to 2,000, preferably from 50 to 500; R₁ is a monovalent radical conforming to the general formula CqH_{2q}L, wherein q is an integer having a value from 2 to 8 and L is selected from the following groups: -N(R₂)CH₂-CH₂-N(R₂)₂; -N(R₂)₂; -N(R₂)₃A; -N(R₂)CHrCH₂-NR₂H₂A; wherein R₂ is hydrogen, phenyl, benzyl, or a saturated hydrocarbon radical, preferably an alkyl radical from about C₁ to about C₂₀, and A⁻ is a halide ion.

A preferred aminosilicone corresponding to formula (XII) is the polymer known as "trimethylsilylamodimethicone", which is shown below in formula (XIII):

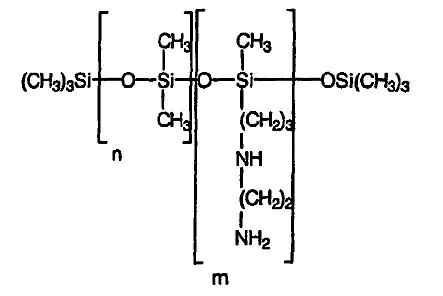

Other aminosilicone polymers which may be used in the compositions of the present invention are represented by the general Formula (XIV): 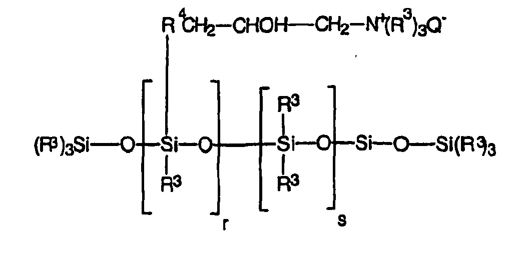 wherein R³ is a monovalent hydrocarbon radical from C₁ to C₁₈, preferably an alkyl or alkenyl radical, such as methyl; R₄ is a hydrocarbon radical, preferably a C₁ to C₁₈ alkylene radical or a C₁₀ to C₁₈ alkyleneoxy radical, more preferably a C₁ to C₈ alkyleneoxy radical; Q⁻ is a halide ion, preferably chloride; r is an average statistical value from 2 to 20, preferably from 2 to 8; s is an average statistical value from 20 to 200, preferably from 20 to 50. A preferred polymer of this class is known as UCARE SILICONE ALE 56™, available from Union Carbide.

### 2. Organopolysiloxane Nanoemulsions

The conditioning agent of the present invention preferably contains an organopolysiloxane in a nanoemulsion comprising polysiloxane particles dispersed in a suitable carrier (typicallly aqueous) with the aid of a surfactant.

Organopolysiloxane nanoemulsions can be produced by the emulsion polymerization of organosiloxane having a low degree of polymerization in a solvent comprising water. The organopolysiloxane is stabilized in the nanoemulsion by a surfactant, e.g., a nonionic surfactant and an ionic surfactant. The average particle size of the emulsion after emulsion polymerization (corresponding to the organopolysiloxane in the emulsion) is less than about 0.1 microns and more preferably less than about 0.06 microns. Particle size of a nanoemulsion can be determined by conventional methods, e.g., using a Leeds & Northrup Microtrac UPA particle sizer. Nanoemulsions having these particle sizes are more stable and have better external appearance than those having larger particle sizes. Furthermore, the degree of polymerization (DP) of the polysiloxane after emulsion polymerization is preferably in the range of from 3 to 5,000, more preferably in the range of from 10 to 3,000.

The organopolysiloxane in the nanoemulsion can be a linear or branched chain siloxane fluid having a viscosity of 20-3,000,000 mm²/s (cs), preferably 300-300,000 cs, more preferably 350-200,000 cs, at 25°C.

Suitable organopolysiloxanes may contain the difunctional repeating "D" unit as in Formula XV: 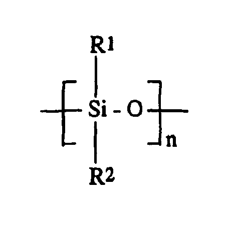 wherein n is greater than 1 and **R¹** and **R²** are each independently C₁-C₇ alkyl or phenyl. A mixture of siloxanes may be used. Exemplary siloxanes include polydimethylsiloxane, polydiethylsiloxane, polymethylethylsiloxane, polymethylphenylsiloxane, and polydiphenylsiloxane. Siloxane polymers with dimethylsiloxane "D" units are preferred from an economic standpoint. However, **R¹** and **R²** may independently be a functional group other than methyl, e.g., carboxyalkyl, haloalkyl, acrylate, acryloxy, acrylamide, vinyl or mercaptoalkyl.

The siloxane may be terminated with hydroxy groups, alkoxy groups such as methoxy, ethoxy, and propoxy, or trimethylsiloxy groups, preferably hydroxy or trimethylsiloxy.

The emulsion can be prepared by the emulsion polymerization process described in EP 459500 (published December 4, 1992). In that process, stable, oil free polysiloxane emulsions and nanoemulsions are prepared by mixing a cyclic siloxane, a nonionic surfactant, an ionic surfactant, water, and a condensation polymerization catalyst. The mixture is heated and agitated at polymerization reaction temperature until essentially all of the cyclic siloxane is reacted, and a stable, oil free emulsion or nanoemulsion is formed. The reaction mix, especially surfactant levels, and conditions are controlled in order to provide the desired organopolysiloxane particle size. The emulsions and nanoemulsions typically have a pH of about 3 to about 10 (e.g., 6-7.5), and contain 10 to 70% by weight siloxane polymer, preferably 25 to 60%, 0% to 30% by weight nonionic surfactant, 0% to 30% by weight ionic surfactant, preferably 0% to 20%, the balance being water. Preferred emulsions and methods of making them are further described in U.S. Patent US 5891954, filed on September 15, 1997 in the names of Ronald P. Gee and Judith M. Vincent.

Nanoemulsions can also be produced by the emulsion polymerization process described in EPA 0268982, published June 6, 1988, assigned to Toray. In this process, the nanoemulsion is prepared by a process in which a crude emulsion, consisting of polysiloxane having a low degree of polymerization, a first surfactant (anionic, cationic, and nonionic surfactants), and water, is slowly dripped into an aqueous solution containing a catalytic quantity of a polymerization catalyst and a second surfactant which acts as an emulsifying agent (which may be the same as the first surfactant, however, the surfactants should be compatible in the reaction mixture considering the ionicity of the reaction mixture). The reaction mix and conditions are controlled to provide the desired organopolysiloxane particle size. Therefore, a dropwise addition of the crude emulsion into the aqueous solution of catalyst and surfactant of 30 minutes or longer is preferred in order to produce nanoemulsions having smaller particle sizes. In addition, the quantity of surfactant used in the catalyst plus the surfactant aqueous solution is from 5 to 70 weight %, more preferably from 25 to 60 per 100 weight parts polysiloxane in the crude emulsion.

Any conventional nonionic surfactant can be used to prepare the nanoemulsion. Exemplary types of nonionic surfactants include silicone polyethers, both grafted and linear block, ethoxylated fatty alcohols, ethoxylated alcohols, ethoxylated alkyl phenols, Isolaureth-6 (polyethylene glycol ether of branched chain aliphatic C₁₂ containing alcohols having the formula C₁₂H₂₅(OCH₂CH₂)₆OH), fatty acid alkanolamides, amine oxides, sorbitan derivatives (e.g., commercially available from ICI Americas, Inc., Wilmington, DE, under the tradenames SPAN and TWEEN), and propylene oxide-ethylene oxide block polymers (e.g., commercially available from BASF Corp., Parsippany, NJ under the trademark PLURONIC). Ionic surfactants useful in preparing the nanoemulsion include any conventional anionic surfactant such as sulfonic acids and their salt derivatives. Ionic surfactants also include any conventional cationic surfactant used in emulsion polymerization. Surfactants of these types are well known in the art and are commercially available from a number of sources. Specific examples of these surfactant types are also disclosed in the above referenced patent application US 5891954.

The surfactant can be used in the form of a single type of surfactant (e.g., anionic, cationic or nonionic), or the surfactant can be used as a combination of two or more types provided that they are compatible with each other and the other components of the composition. Preferred combinations of surfactant types include the combination of two or more types of anionic surfactants, the combination of two or more types of nonionic surfactants, the combination of two or more types of cationic surfactants, the combination of two or more types of surfactants selected from both the anionic and nonionic surfactants; and the combination of two or more types of surfactants selected from both the cationic and nonionic surfactants.

The catalyst employed in the emulsion polymerization may be any catalyst capable of polymerizing cyclic siloxanes in the presence of water, including condensation polymerization catalysts capable of cleaving siloxane bonds. Exemplary catalysts include strong acids and strong bases, ionic surfactants such as dodecylbenzenesulfonic acid, phase transfer catalysts, and ion exchange resins where a catalyst is formed *in situ.* As will be understood by those skilled in the art, a given surfactant may also serve as the polymerization catalyst (e.g., alkylbenzenesulfonic acids, or quaternary ammonium hydroxides or salt thereof may function as both a surfactant and the polymerization catalyst).

A surfactant system, catalyst and resulting nanoemulsion suitable for use in the compositions of the present invention can be selected by the skilled artisan considering the ionicity of the composition. In general, these materials are selected such that the total composition will be compatible.

The silicone nanoemulsion may contain a silicone polyether copolyol, such as described herein. Alternatively or additionally, the compositions hereof may contain a silicone polyether. If the nanoemulsion is supplied as a material not containing a silicone polyether already present in the emulsion, a silicone polyether may be added prior to making the batch composition hereof. Where the polyether is not dispersible in the silicone nanoemulsion, it is preferably mixed in about an equal portion of water containing from 10-50% C₁-C₃ monohydric alcohol, preferably ethanol, prior to combination with the silicone nanoemulsion. This pre-mix is then added to the other ingredients of the composition which have preferably been pre-mixed.

Organopolysiloxane nanoemulsions are available from a number of commercial sources.

The following organopolysiloxane nanoemulsions are manufactured by Dow Corning of Midland, MI:

**Nanoemulsions not containing dimethicone copolyol:**

| Nanoemulsion Trade Name | Si Type | Internal phase viscosity (cps) | Surfactant | Si particle, size, nm |
|---|---|---|---|---|
| DC 2-1470 | Dimethylsiloxanol, Dimethyl cyclosiloxane | 15-20M | Triethanolamine dodecylbenzene sulfonate, Polyethylene oxide lauryl ether | <50 |
| DC 2-1845 | Dimethylsiloxanol, Dimethyl cyclosiloxane | 4-8M | Triethanolamine dodecylbenzene sulfonate, Polyethylene oxide lauryl ether | <40 |
| DC 2-1845-HV | Dimethylsiloxanol, Dimethyl cyclosiloxane | 60-70M | Triethanolamine dodecylbenzene sulfonate, Polyethylene oxide lauryl ether | <35 |
| DC 2-1550 | Dimethylsiloxanol, Dimethyl cyclosiloxane | 100-600M | Triethanolamine dodecylbenzene sulfonate, Polyethylene oxide lauryl ether | ≤50 |
| DC 2-1281 | Dimethylsiloxanol, Dimethyl cyclosiloxane | 1-2M | Cetrimonium Chloride, Trideceth-12 | <30 |
| DC 2-8194 | Dimethyl, aminomethyl propyl siloxane | 4-6M | Cetrimonium Chloride, Trideceth-12 | <30 |
| DC 2-1716 MEM | Dimethylsiloxanol with methyl silsequioxane, Octamethyl cyclotretrasiloxane | 10-30M | Cetrimonium Chloride, Trideceth-12 | 50-80 |

| | | | | |
|---|---|---|---|---|
| A particularly preferred nanoemulsion is DC-2-1550 | | | | |

**Nanoemulsions containing dimethicone copolyol:**

| Nanoemulsion Trade Name | Si Type | Internal phase viscosity (cps) | Surfactant | Si particle, size, nm |
|---|---|---|---|---|
| DC 2-5791 | Dimethylsiloxanol, Dimethyl cyclosiloxane | 70-90M | Triethanolamine dodecylbenzene sulfonate, Polyethylene oxide lauryl ether | <50 |
| DC 2-5791-sp | Dimethylsiloxanol, Dimethyl cyclosiloxane | 70-90M | Triethanolamine dodecylbenzene sulfonate, Polyethylene oxide lauryl ether | <40 |
| DC 2-5932 | Dimethylsiloxanol, Dimethyl cyclosiloxane | 1-2M | Cetrimonium Chloride, Trideceth-12 | <30 |

### 3. Organic conditioning oils

The nanoemulsion conditioning component of the personal cleansing compositions of the present invention may also comprise from 0.05% to 3%, by weight of the composition, preferably from 0.08% to 1.5%, more preferably from 0.1% to 1%, of at least one organic conditioning oil as the conditioning agent, either alone or in combination with other conditioning agents, such as the silicones (described above).

### a. Hydrocarbon oils

Suitable organic conditioning oils for use as conditioning agents in the personal cleansing compositions of the present invention include, but are not limited to, hydrocarbon oils having at least about 10 carbon atoms, such as cyclic hydrocarbons, straight chain aliphatic hydrocarbons (saturated or unsaturated), and branched chain aliphatic hydrocarbons (saturated or unsaturated), including polymers and mixtures thereof. Straight chain hydrocarbon oils preferably are from about C₁₂ to about C₁₉. Branched chain hydrocarbon oils, including hydrocarbon polymers, typically will contain more than 19 carbon atoms.

Specific non-limiting examples of these hydrocarbons oils include paraffin oil, mineral oil, saturated and unsaturated dodecane, saturated and unsaturated tridecane, saturated and unsaturated tetradecane, saturated and unsaturated pentadecane, saturated and unsaturated hexadecane, polybutene, polydecene, and mixtures thereof. Branched-chain isomers of these compounds, as well as of higher chain length hydrocarbons, can also be used, examples of which include highly branched, saturated or unsaturated, alkanes such as the permethyl-substituted isomers, e.g., the permethyl-substituted isomers of hexadecane and eicosane, such as 2, 2, 4, 4, 6, 6, 8, 8-dimethyl-10-methylundecane and 2, 2, 4, 4, 6, 6-dimethyl-8-methylnonane, available from Permethyl Corporation. Hydrocarbon polymers such as polybutene and polydecene. A preferred hydrocarbon polymer is polybutene, such as the copolymer of isobutylene and butene. A commercially available material of this type is L-14 polybutene from Amoco Chemical Corporation.

### b. Polyolefins

Organic conditioning oils for use in the personal cleansing compositions of the present invention can also include liquid polyolefins, more preferably liquid poly-α-olefins, more preferably hydrogenated liquid poly-α-olefins. Polyolefins for use herein are prepared by polymerization of C₄ to about C₁₄ olefenic monomers, preferably from about C₆ to about C₁₂.

Non-limiting examples of olefenic monomers for use in preparing the polyolefin liquids herein include ethylene, propylene, 1-butene, 1-pentene, 1-hexene, 1-octene, 1-decene, 1-dodecene, 1-tetradecene, branched chain isomers such as 4-methyl-1-pentene and mixtures thereof. Also suitable for preparing the polyolefin liquids are olefin-containing refinery feedstocks or effluents. Preferred hydrogenated α-olefin monomers include, but are not limited to: 1-hexene to 1-hexadecenes, 1-octene to 1-tetradecene, and mixtures thereof.

### c. Fatty Esters

Other suitable organic conditioning oils for use as the conditioning agent in the personal cleansing compositions of the present invention include, but are not limited to, fatty esters having at least 10 carbon atoms. These fatty esters include esters with hydrocarbyl chains derived from fatty acids or alcohols (e.g. mono-esters, polyhydric alcohol esters, and di- and tri-carboxylic acid esters). The hydrocarbyl radicals of the fatty esters hereof may include or have covalently bonded thereto other compatible functionalities, such as amides and alkoxy moieties (e.g., ethoxy or ether linkages, etc.).

Specific examples of preferred fatty esters include, but are not limited to: isopropyl isostearate, hexyl laurate, isohexyl laurate, isohexyl palmitate, isopropyl palmitate, decyl oleate, isodecyl oleate, hexadecyl stearate, decyl stearate, isopropyl isostearate, dihexyldecyl adipate, lauryl lactate, myristyl lactate, cetyl lactate, oleyl stearate, oleyl oleate, oleyl myristate, lauryl acetate, cetyl propionate, and oleyl adipate.

Other fatty esters suitable for use in the personal cleansing compositions of the present invention are mono-carboxylic acid esters of the general formula R'COOR, wherein R' and R are alkyl or alkenyl radicals, and the sum of carbon atoms in R' and R is at least 10, preferably at least 22.

Still other fatty esters suitable for use in the personal cleansing compositions of the present invention are di- and tri-alkyl and alkenyl esters of carboxylic acids, such as esters of C₄ to C₈ dicarboxylic acids (e.g. C₁ to Cn esters, preferably C₁ to C₆, of succinic acid, glutaric acid, adipic acid,). Specific non-limiting examples of di- and tri- alkyl and alkenyl esters of carboxylic acids include isocetyl stearyol stearate, diisopropyl adipate, and tristearyl citrate.

Other fatty esters suitable for use in the personal cleansing compositions of the present invention are those known as polyhydric alcohol esters. Such polyhydric alcohol esters include alkylene glycol esters, such as ethylene glycol mono and di-fatty acid esters, diethylene glycol mono- and di-fatty acid esters, polyethylene glycol mono- and di-fatty acid esters, propylene glycol mono- and di-fatty acid esters, polypropylene glycol monooleate, polypropylene glycol 2000 monostearate, ethoxylated propylene glycol monostearate, glyceryl mono- and di-fatty acid esters, polyglycerol poly-fatty acid esters, ethoxylated glyceryl monostearate, 1,3-butylene glycol monostearate, 1,3-butylene glycol distearate, polyoxyethylene polyol fatty acid ester, sorbitan fatty acid esters, and polyoxyethylene sorbitan fatty acid esters.

Still other fatty esters suitable for use in the personal cleansing compositions of the present invention are glycerides, including, but not limited to, mono-, di-, and tri-glycerides, preferably di- and tri-glycerides, more preferably triglycerides. For use in the personal cleansing compositions described herein, the glycerides are preferably the mono-, di-, and tri-esters of glycerol and long chain carboxylic acids, such as C₁₀ to C₂₂ carboxylic acids. A variety of these types of materials can be obtained from vegetable and animal fats and oils, such as castor oil, safflower oil, cottonseed oil, corn oil, olive oil, cod liver oil, almond oil, avocado oil, palm oil, sesame oil, lanolin and soybean oil. Synthetic oils include, but are not limited to, triolein and tristearin glyceryl dilaurate.

Other fatty esters suitable for use in the personal cleansing compositions of the present invention are water insoluble synthetic fatty esters. Some preferred synthetic esters conform to the general Formula (XVI): 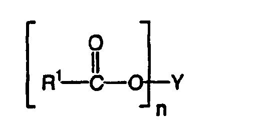 wherein R¹ is a C₇ to C₉ alkyl, alkenyl, hydroxyalkyl or hydroxyalkenyl group, preferably a saturated alkyl group, more preferably a saturated, linear, alkyl group; n is a positive integer having a value from 2 to 4, preferably 3; and Y is an alkyl, alkenyl, hydroxy or carboxy substituted alkyl or alkenyl, having from about 2 to about 20 carbon atoms, preferably from about 3 to about 14 carbon atoms. Other preferred synthetic esters conform to the general Formula (XVII): 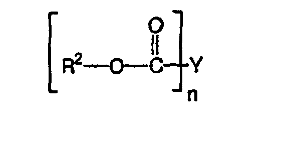 wherein R² is a C₈ to C₁₀ alkyl alkenyl, hydroxyalkyl or hydroxyalkenyl group; preferably a saturated alkyl group, more preferably a saturated, linear, alkyl group; n and Y are as defined above in Formula (XVII).

Specific non-limiting examples of suitable synthetic fatty esters for use in the personal cleansing compositions of the present invention include: P-43 (C₈-C₁₀ triester of trimethylolpropane), MCP-684 (tetraester of 3,3 diethanol-1,5 pentadiol), MCP 121 (C₈-C₁₀ diester of adipic acid), all of which are available from Mobil Chemical Company.

### 3. Other conditioning agents

Also suitable for use in the compositions herein are the conditioning agents described by the Procter & Gamble Company in U.S. Pat. Nos. 5,674,478, and 5,750,122. Also suitable for use herein are those conditioning agents described in U.S. Pat: Nos. 4,529,586 (Clairol), 4,507,280 (Clairol), 4,663,158 (Clairol), 4,197,865 (L'Oreal), 4,217, 914 (L'Oreal), 4,381,919 (L'Oreal), and 4,422, 853 (L'Oreal).

### Additional Components

### Suspending agent

The shampoo compositions of the present invention may further comprise a suspending agent at concentrations effective for suspending the polymeric liquid crystal or the dispersed particles of a water insoluble, conditioning agent, or other water-insoluble, dispersed material in the shampoo compositions. Such concentrations range from 0.1% to 10%, preferably from 0.3% to 5.0%, by weight of the shampoo compositions.

Suitable suspending agents include crystalline suspending agents that can be categorized as acyl derivatives, long chain amine oxides, or combinations thereof. These suspending agents are described in U.S. Patent 4,741,855. These preferred suspending agents include ethylene glycol esters of fatty acids preferably having from 16 to 22 carbon atoms. More preferred are the ethylene glycol stearates, both mono and distearate, but particularly the distearate containing less than 7% of the mono stearate. Other suitable suspending agents include alkanol amides of fatty acids, preferably having from about 16 to about 22 carbon atoms, more preferably about 16 to 18 carbon atoms, preferred examples of which include stearic monoethanolamide, stearic diethanolamide, stearic monoisopropanolamide and stearic monoethanolamide stearate. Other long chain acyl derivatives include long chain esters of long chain fatty acids (e.g., stearyl stearate, cetyl palmitate, etc.); glyceryl esters (e.g., glyceryl distearate) and long chain esters of long chain alkanol amides (e.g., stearamide diethanolamide distearate, stearamide monoethanolamide stearate). Long chain acyl derivatives, ethylene glycol esters of long chain carboxylic acids, long chain amine oxides, and alkanol amides of long chain carboxylic acids in addition to the preferred materials listed above may be used as suspending agents. For example, it is contemplated that suspending agents with long chain hydrocarbyl having C₈-C₂₂ chains may be used.

Other long chain acyl derivatives suitable for use as suspending agents include N,N-dihydrocarbyl amido benzoic acid and soluble salts thereof (e.g., Na, K), particularly N,N-di(hydmgenated) C₁₆, C₁₈ and tallow amido benzoic acid species of this family, which are commercially available from Stepan Company (Northfield, Illinois, USA).

Examples of suitable long chain amine oxides for use as suspending agents include alkyl (C₁₆-C₂₂) dimethyl amine oxides, e.g., stearyl dimethyl amine oxide

Other suitable suspending agents include primary amines having a fatty alkyl moiety having at least about 16 carbon atoms, examples of which include palmitamine or stearamine, and secondary amines having two fatty alkyl moieties each having at least about 12 carbon atoms, examples of which include dipalmitoylamine or di(hydrogenated tallow)amine. Still other suitable suspending agents include di(hydrogenated tallow)phthalic acid amide, and crosslinked maleic anhydride-methyl vinyl ether copolymer.

### Dispersed Particles

The composition of the present invention may include dispersed particles. In the compositions of the present invention, it is preferable to incorporate at least 0.025% by weight of the dispersed particles, more preferably at least 0.05%, still more preferably at least 0.1%, even more preferably at least 0.25%, and yet more preferably at least 0.5% by weight of the dispersed particles. In the compositions of the present invention, it is preferable to incorporate no more than 20% by weight of the dispersed particles, more preferably no more than 10%, still more preferably no more than 5%, even more preferably no more than 3%, and yet more preferably no more than 2% by weight of the dispersed particles.

### Anti-dandruff Actives

The compositions of the present invention may also contain an anti-dandruff agent Suitable, non-limiting examples of anti-dandruff particulates include: pyridinethione salts, azoles, selenium sulfide, particulate sulfur, and mixtures thereof. Preferred are pyridinethione salts. Such anti-dandruff particulate should be physically and chemically compatible with the essential components of the composition, and should not otherwise unduly impair product stability, aesthetics or performance.

### Pyridinethione salts

Pyridinethione anti-dandruff particulates, especially 1-hydroxy-2-pyridinethione salts, are highly preferred particulate anti-dandruff agents for use in compositions of the present invention. The concentration of pyridinethione anti-dandruff particulate typically ranges from 0.1% to 4%, by weight of the composition, preferably from 0.1% to 3%, more preferably from 0.3% to 2%. Preferred pyridinethione salts include those formed from heavy metals such as zinc, tin, cadmium, magnesium, aluminum and zirconium, preferably zinc, more preferably the zinc salt of 1-hydroxy-2-pyridinethione (known as "zinc pyridinethione" or "ZPT"), more preferably 1-hydroxy-2-pyridinethione salts in platelet particle form, wherein the particles have an average size of up to about 20µ, preferably up to about 5µ, more preferably up to about 2.5µ. Salts formed from other cations, such as sodium, may also be suitable. Pyridinethione anti-dandruff agents are described, for example, in U.S. Pat. No. 2,809,971; U.S. Pat. No. 3,236,733; U.S. Pat. No. 3,753,196; U.S. Pat. No. 3,761,418; U.S. Pat. No. 4,345,080; U.S. Pat. No. 4,323,683; U.S. Pat. No. 4,379,753; and U.S. Pat. No. 4,470,982. It is contemplated that when ZPT is used as the anti-dandruff particulate in the compositions herein, that the growth or re-growth of hair may be stimulated or regulated, or both, or that hair loss may be reduced or inhibited, or that hair may appear thicker or fuller.

**Other Anti-microbial Actives** - In addition to the anti-dandruff active selected from polyvalent metal salts of pyrithione, the present invention may further comprise one or more antifungal or anti-microbial actives in addition to the metal pyrithione salt actives. Suitable anti-microbial actives include coal tar, sulfur, whitfield's ointment, castellani's paint, aluminum chloride, gentian violet, octopirox (piroctone olamine), ciclopirox olamine, undecylenic acid and it's metal salts, potassium permanganate, selenium sulphide, sodium thiosulfate, propylene glycol, oil of bitter orange, urea preparations, griseofulvin, 8-Hydroxyquinoline ciloquinol, thiobendazole, thiocarbamates, haloprogin, polyenes, hydroxypyridone, morpholine, benzylamine, allylamines (such as terbinafine), tea tree oil, clove leaf oil, coriander, palmarosa, berberine, thyme red, cinnamon oil, cinnamic aldehyde, citronellic acid, hinokitol, ichthyol pale, Sensiva SC-50, Elestab HP-100, azelaic acid, lyticase, iodopropynyl butylcarbamate (IPBC), isothiazalinones such as octyl isothiazalinone and azoles, and combinations thereof. Preferred anti-microbials include itraconazole, ketoconazole, selenium sulphide and coal tar.

### Azoles

Azole anti-microbials include imidazoles such as benzimidazole, benzothiazole, bifonazole, butaconazole nitrate, climbazole, clotrimazole, croconazole, eberconazole, econazole, elubiol, fenticonazole, fluconazole, flutimazole, isoconazole, ketoconazole, lanoconazole, metronidazole, miconazole, neticonazole, omoconazole, oxiconazole nitrate, sertaconazole, sulconazole nitrate, tioconazole, thiazole, and triazoles such as terconazole and itraconazole, and combinations thereof. When present in the composition, the azole anti-microbial active is included in an amount from 0.01% to 5%, preferably from 0.1% to 3%, and more preferably from 0.3% to 2%, by weight of the composition. Especially preferred herein is ketoconazole.

### Selenium Sulfide

Selenium sulfide is a particulate anti-dandruff agent suitable for use in the anti-microbial compositions of the present invention, effective concentrations of which range from 0.1% to 4%, by weight of the composition, preferably from 0.3% to 2.5%, more preferably from 0.5% to 1.5%. Selenium sulfide is generally regarded as a compound having one mole of selenium and two moles of sulfur, although it may also be a cyclic structure that conforms to the general formula SeₓS_{y}, wherein x + y = 8. Average particle diameters for the selenium sulfide are typically less than 15µm, as measured by forward laser light scattering device (e.g. Malvern 3600 instmment), preferably less than 10 µm. Selenium sulfide compounds are described, for example, in U.S. Pat. No. 2,694,668; U.S. Pat. No. 3,152,046; U.S. Pat. No. 4,089,945; and U.S. Pat. No. 4,885,107.

### Sulfur

Sulfur may also be used as a particulate anti-microbial/anti-dandruff agent in the anti-microbial compositions of the present invention. Effective concentrations of the particulate sulfur are typically from 1% to 4%, by weight of the composition, preferably from 2% to 4%.

### Keratolytic Agents

The present invention may further comprise one or more keratolytic agents such as Salicylic Acid.

Additional anti-microbial actives of the present invention may include extracts of melaleuca (tea tree) and charcoal. The present invention may also comprise combinations of anti-microbial actives. Such combinations may include octopirox and zinc pyrithione combinations, pine tar and sulfur combinations, salicylic acid and zinc pyrithione combinations, octopirox and climbasole combinations, and salicylic acid and octopirox combinations, and mixtures thereof.

### Humectant

The compositions of the present invention may contain a humectant. The humectants herein are selected from the group consisting of polyhydric alcohols, water soluble alkoxylated nonionic polymers, and mixtures thereof. The humectants, when used herein, are preferably used at levels by weight of the composition of from 0.1% to 20%, more preferably from 0.5% to 5%.

Polyhydric alcohols useful herein include glycerin, sorbitol, propylene glycol, butylene glycol, hexylene glycol, ethoxylated glucose, 1, 2-hexane diol, hexanetriol, dipropylene glycol, erythritol, trehalose, diglycerin, xylitol, maltitol, maltose, glucose, fructose, sodium chondroitin sulfate, sodium hyaluronate, sodium adenosine phosphate, sodium lactate, pyrrolidone carbonate, glucosamine, cyclodextrin, and mixtures thereof.

Water soluble alkoxylated nonionic polymers useful herein include polyethylene glycols and polypropylene glycols having a molecular weight of up to about 1000 such as those with CTFA names PEG-200, PEG-400, PEG-600, PEG-1000, and mixtures thereof.

### Other Optional Components

The compositions of the present invention may contain also vitamins and amino acids such as: water soluble vitamins such as vitamin B1, B2, B6, B12, C, pantothenic acid, pantothenyl ethyl ether, panthenol, biotin, and their derivatives, water soluble amino acids such as asparagine, alanin, indole, glutamic acid and their salts, water insoluble vitamins such as vitamin A, D, E, and their derivatives, water insoluble amino acids such as tyrosine, tryptamine, and their salts.

The compositions of the present invention may also contain pigment materials such as inorganic, nitroso, monoazo, disazo, carotenoid, triphenyl methane, triaryl methane, xanthene, quinoline, oxazine, azine, anthraquinone, indigoid, thionindigoid, quinacridone, phthalocianine, botanical, natural colors, including: water soluble components such as those having C. L Names.

The compositions of the present invention may also contain antimicrobial agents which are useful as cosmetic biocides and antidandruff agents including: water soluble components such as piroctone olamine, water insoluble components such as 3,4,4'- trichlorocarbanilide (trichlosan), triclocarban and zinc pyrithione. The compositions of the present invention may also contain chelating agents.

### Method of Manufacture

The compositions of the present invention, in general, may be made by mixing together at elevated temperature, e.g., about 72.degree. C. water and surfactants along with any solids (e.g. amphiphiles) that need to be melted, to speed mixing into the personal cleansing composition. The ingredients are mixed thoroughly at the elevated temperature and then cooled to ambient temperature. Additional ingredients, including electrolytes, polymers, and particles, may be added to the cooled product. The conditioning agent may be emulsified at room temperature in concentrated surfactant to form a nanoemulsion or purchased as a pre-formed nanoemulsion material and then added to the cooled product.

### Method of Use

The shampoo compositions of the present invention are used in a conventional manner for cleaning and conditioning hair or skin. An effective amount of the composition for cleansing and conditioning the hair or skin is applied to the hair or skin, that has preferably been wetted with water, and then rinsed off. Such effective amounts generally range from about 1gm to about 50gm, preferably from about 1gm to about 20gm. Application to the hair typically includes working the composition through the hair such that most or all of the hair is contacted with the composition.

This method for cleansing and conditioning the hair or skin comprises the steps of:
a) wetting the hair or skin with water, b) applying an effective amount of the shampoo composition to the hair or skin, and c) rinsing the applied areas of skin or hair with water. These steps can be repeated as many times as desired to achieve the desired cleansing and conditioning benefit.

### Non-limiting Examples

The compositions illustrated in the following Examples illustrate specific embodiments of the compositions of the present invention. These exemplified embodiments of the composition of the present invention provide enhanced conditioning benefits to the hair.

The compositions illustrated in the following Examples are prepared by conventional formulation and mixing methods, an example of which is described above. All exemplified amounts are listed as weight percents and exclude minor materials such as diluents, preservatives, color solutions, imagery ingredients, botanicals, and so forth, unless otherwise specified.

The following table demonstrates the increased deposition of conditioning actives resulting from using preferred polymers of the celullosic type. The data demonstrate increased deposition of conditioning active with increase in charge density.

The data were obtained utilizing a silicone deposition test, wherein the products to be tested are applied to hair switches under consumer relevant conditions. After rinsing, the switches are dried and extracted in an appropriate solvent. The extracts are then introduced into an atomic absorption/emission detector set at the appropriate wavelength. The absorbance/emission value returned by the instrument is then converted to actual concentration of material on hair (ug/g or ppm) through an external calibration curve obtained with known weights of a well characterized standard of the material under study. Blanks are also ran.

| Polymer | CD meq/gm | MW | Silicone (1) | |
|---|---|---|---|---|
| | | | | |
| Polyquaternium 10 (2) | 0.6638 | 700,000 | 264 | |
| Polyquaternium 10 (2) | 1.492 | 700,000 | 1192 | Preferred |
| Polyquaternium 10 (2) | 0.7066 | 1,750,000 | 275 | |
| Polyquaternium 10 (2) | 1.478 | 1,750,000 | 1055 | Preferred |
| | | | | |
| Polyquaternium 10 (2) | 0.7 | 1,300,000 | 122 | |
| Polymer JR30M (3) | 1.25 | 1,300,000 | 380 | Taught in EP 529 883A1 |
| Polyquaternium 10 (2) | 1.4 | 1,300,000 | 555 | Preferred |
| Polyquaternium 10 (2) | 1.9 | 1,300,000 | 738 | Highly Preferred |

| | | | | |
|---|---|---|---|---|
| (1) Polydimethylsiloxane with a volume average particle size of 0.3 microns (2) All polymers are available from Amerchol/Dow (3) Polymer JR30M, INCI name: Polyquaternium 10 also available from Amerchol/Dow | | | | |

The following table demonstrates the increased deposition of conditioning actives preferred polymers of the guar type, the data clearly demonstrating the increased deposition of conditioning active with increase in charge density. In the table there are four distinctly different molecular weight ranges, low, medium, medium-high and high. For each molecular weight, polymers were synthesized with as close as possible molecular weights, but varying charge density. These data clearly indicate that higher charge densities cationic polymers yield higher conditioning agent deposition and hence are more preferred versus those taught in the art. The data was generated using the silicone deposition test described hereinbefore.
All polymers are Guar Hydroxypropyl trimonium chloride

| MW range | MW x1000 | CD | Silicone (1) | |
|---|---|---|---|---|
| Low (2) | 59 | 0.76 | 254 | |
| Low (2) | 65 | 1.11 | 399 | |
| Low (2) | 62 | 2.05 | 880 | Preferred |
| | | | | |
| Medium (2) | 550 | 0.76 | 637 | |
| Medium (2) | 524 | 2.01 | 1941 | Highly Preferred |
| | | | | |
| Medium-High (2) | 972 | 0.76 | 669 | |
| Medium-High (2) | 979 | 2.05 | 2743 | Highly Preferred |
| | | | | |
| High (2) | 1,310 | 1.11 | 532 | |
| High (2) | 1330 | 2.01 | 1233 | Highly preferred |
| | | | | |
| Jaguar C-13S (3) | 2,200,000 | 0.5 | 373 | |
| Jaguar C-17 (4) | 2,200,000 | 0.9 | 835 | |

| | | | | |
|---|---|---|---|---|
| (1) Polydimethylsiloxane with a volume average particle size of 0.3 microns (2) Guar Hydroxypropyl trimonium chloride all polymers are available from Aqualon (3) Guar Hydroxypropyl trimonium chloride, data generated on equivalent charge density and molecular weight guar synthesized by Aqualon (4) Guar Hydroxypropyl trimonium chloride, Jaguar C-17 available from Rhodia | | | | |

| | **Example 1** | **Example 2** | **Example 3** | **Example 4** | **Example 5** | **Example 6** | **Example 7** |
|---|---|---|---|---|---|---|---|
| Ammonium Laureth Sulfate | | | 14.00 | 14.00 | | 14.00 | 14.00 |
| Sodium Laureth Sulfate | 12.00 | 12.00 | | | 12.00 | | |
| Sodium Lauryl Sulfate | 2.00 | 2.00 | | | 2.00 | | |
| Cocamidopropyl Betaine | 2.00 | 2.00 | 2.70 | 2.70 | 2.00 | 2.70 | 2.70 |
| Sodium Lauroamphoacetate | 2.00 | 2.00 | | | 2.00 | | |
| Ethylene Glycol Distearate | 1.50 | 1.50 | 1.50 | 1.50 | 1.50 | 1.50 | 1.50 |
| CMEA | 0.800 | 0.800 | 0.800 | 0.800 | 0.800 | 0.800 | 0.800 |
| Cetyl Alcohol | 0.900 | 0.900 | 0.600 | 0.600 | 0.900 | 0.600 | 0.600 |
| Lauryl Alcohol | 0.200 | 0.200 | | | 0.200 | | |
| Guar Hydroxypropyl Trimonium Chloride (1) | 0.750 | | | | 0.250 | | |
| Guar Hydroxypropyl Trimonium Chloride (2) | | 0.500 | | | | | |
| Guar Hydroxypropyl Trimonium Chloride (3) | | | 0.500 | | | | |
| Polyquaterium-10 (4) | | | | 0.500 | | | |
| Polyquaterium-10 (5) | | | | | 0.500 | | |
| Polyquaterium-10 (6) | | | | | | 0.500 | |
| Polyquaterium-10 (7) | | | | | | | 0.500 |
| | | | | | | | |
| Dimethicone (8) | 1.00 | 1.00 | 1.00 | 1.00 | 1.00 | 1.00 | 1.00 |
| Hydrogenated Polydecene (9) | | | 0.25 | | | | |
| Sodium Citrate | | | 0.40 | 0.40 | | 0.40 | 0.40 |
| Citric Acid | | | 0.39 | 0.39 | | 0.39 | 0.39 |
| Hydrochloric Acid | 0.600 | 0.600 | 0.600 | 0.600 | 0.600 | 0.600 | 0.600 |
| Sodium Xylenesulfonate | 1.00 | 1.00 | 1.00 | 1.00 | 1.00 | 1.00 | 1.00 |
| Perfume | 0.400 | 0.400 | 0.400 | 0.400 | 0.400 | 0.400 | 0.400 |
| Sodium Benzoate | 0.250 | 0.250 | 0.250 | 0.250 | 0.250 | 0.250 | 0.250 |
| Kathon | 0.0008 | 0.0008 | 0.0008 | 0.0008 | 0.0008 | 0.0008 | 0.0008 |
| Benzyl Alcohol | 0.0225 | 0.0225 | 0.0225 | 0.0225 | 0.0225 | 0.0225 | 0.0225 |
| Water | q.s. | q.s. | q.s. | q.s. | q.s. | q.s. | q.s. |

| | | | | | | | |
|---|---|---|---|---|---|---|---|
| 1. Guar having a molecular weight of about 600,000, and having a charge density of about 2.40 meq/g, available from Aqualon. 2. Guar having a molecular weight of about 1,100,000, and having a charge density of about 2.10 meq/g, available from Aqualon. 3. Guar having a molecular weight of about 400,000, and having a charge density of about 1.57 meq/g, available from Aqualon. 4. Polymer KG30M available from Amerchol/Dow with a charge density of 1.97 meq/gm and a molecular weight of 2,000,000 5. Cationic cellulose polymer available from Amerchol/Dow with a charge density of 2.38 meq/gm and a molecular weight of 2,000,000 6. Cationic cellulose polymer available from Amerchol/Dow with a charge density of 1.79 meq/gm and a molecular weight of 2,000,000 7. Cationic cellulose polymer available from Amerchol/Dow with a charge density of 2.74 meq/gm and a molecular weight of 450,000 8. DC1664 available from Dow Corning Silicones with a particle size of 0.3 microns 9. Mobil P43, available from Mobil. | | | | | | | |

| | **Example 8** | **Example 9** | **Example 10** | **Example 11** | **Example 12** | **Example 13** |
|---|---|---|---|---|---|---|
| Sodium Laureth Sulfate | 15.00 | 15.00 | 10.00 | 15.00 | 15.00 | 10.00 |
| Sodium Lauryl Sulfate | 5.00 | 5.00 | 6.00 | 5.00 | 5.00 | 6.00 |
| CMEA | 0.800 | 0.800 | 0.800 | 0.800 | 0.800 | 0.800 |
| Polyquaternium 10 (1) | 0.250 | 0.250 | 0.500 | 0.250 | | |
| Guar Hydroxypropyl trimonium | | | | | 0.500 | 0.500 |
| chloride (2) | | | | | | |
| Dimethylsiloxanol, Dimethyl cyclosiloxane (3) | 0.500 | | | | 0.500 | |
| Dimethylsiloxanol, Dimethyl cyclosiloxane (4) | | 1.00 | | | | 0.250 |
| Dimethyl, aminomethyl propyl siloxane (5) | | | 0.250 | | | |
| Dimethylsiloxanol, Dimethyl cyclosiloxane (6) | | | | 0.500 | | |
| | | | | | | |
| Sodium Citrate | 0.400 | 0.400 | 0.200 | 0.400 | 0.400 | 0.200 |
| Citric Acid | 0.2200 | 0.2200 | 0.2200 | 0.2200 | 0.3300 | 0.3300 |
| Sodium Chloride | 1.000 | 1.000 | 1.000 | 1.000 | 1.000 | 1.000 |
| Perfume | 0.550 | 0.550 | 0.550 | 0.550 | 0.700 | 0.700 |
| Sodium Benzoate | | | 0.250 | 0.250 | | |
| Ethylene Diamine Tetra Acetic Acid | 0.12 | 0.12 | 0.12 | 0.12 | 0.12 | 0.12 |
| Kathon | 0.0005 | 0.0005 | 0.0005 | 0.0005 | 0.0005 | 0.0005 |
| Water | q.s. | q.s. | q.s. | q.s. | q.s. | q.s. |

| | | | | | | |
|---|---|---|---|---|---|---|
| 1. Polymer KG30M available from Amerchol/Dow with a charge density of 1.97 meq/gm and a molecular weight of 2,000,000 2. Guar having a molecular weight of about 400,000, and having a charge density of about 2.10 meq/g, available from Aqualon. 3. DC-2-1550 silicone nanoemulsion from Dow Corning with a particle size of less than 50 nm, an anionic/nonionic surfactant system, and a silicone with an internal phase viscosity= 100,000 cps 4. DC-1845 - Silicone nanoemulsion from Dow Corning with a particle size of 33 nm, an anionic/nonionic surfactant system, and a silicone with an internal phase viscosity = 70,000 cps. 5. DC-2-8194 - Aminosilicone nanoemulsion from Dow Corning with a particle size of 30 nm, a cationic surfactant system, and a silicone with an internal phase visocosity = 4000 cps. 6. DC 2-5791 SP - Silicone nanoemulsion from Dow Corning with a particle size of 37 nm, an anionic/nonionic surfactant system, and a silicone with an internal phase viscosity = 90,000 cps. | | | | | | |

## Claims

1. A shampoo composition comprising:
a) from 4 to 50 weight percent of a detersive surfactant comprising anionic surfactant and betaines,
b) at least 0.05 weight percent of a cationic polymer,
i) wherein said cationic polymer has a molecular weight from 10,000 to 10,000, 000; and
ii) wherein said cationic polymer has a charge density from 1.4meq/g to 7meq/g,
c) at least 0.05 weight percent of a conditioning agent material having a volume average diameter of less than 1.0 microns; and
d) at least 20. 0 weight percent of an aqueous carrier.

2. The composition of claim 1 wherein said cationic polymer is a cationic polysaccharide.

3. The composition of claim 2 wherein said cationic polysaccharide is selected from the group consisting of cationic anhydroglucoses, cationic guar derivatives, cationic starches, and cationic celluloses.

4. The composition of claim 1 wherein said conditioning agent material contains silicone.

5. The composition of claim 4 wherein the said silicone conditioning agent material is an organosiloxane nanoemulsion.

6. The composition of claim 5 wherein the said organosiloxane nanoemulsion has a volume average diameter of less than 0.1 microns.

7. The composition of claim1 wherein said conditioning agent material has a volume average diameter of less than 0.3 microns, preferably less than 0.1 microns, more preferably of less than 05 microns.

8. The composition of claim 1 wherein said cationic polymer has a molecular weight of from 50,000 to 5,000, 000, preferably from 100,000 to 3,000, 000.

9. The composition of claim 1 wherein said cationic polymer has a charge density of at least 1.6 meq/gm, preferably at least 1.8meq/gm, most preferably at least 2.0meq/gm.

10. The composition of claim 1 wherein said cationic polymer has a charge density of less than 4meq/gm.

11. The composition of claim 1 wherein the weight ratio of said cationic polymer to said conditioning agent is from 4: 1 to 1: 40, preferably from 2:1 to 1: 20, more preferably from 1: 1 to 1: 5.

12. The composition of claim 1 comprising from 0.075 weight percent to 2 weight percent of said cationic polymer preferably from 0.1 weight percent to 1 weight percent of said cationic polymer.

13. The composition of claim 1 comprising from 0.1 weight percent to 5 weight percent, preferably from 0.2 weight percent to 3 weight percent of said conditioning agent.

14. A cosmetic method of treating hair by administering a safe and effective amount of the composition according to claim 1.

## Patentansprüche

1. Shampoozusammensetzung, umfassend:
a) von 4 bis 50 Gewichtsprozent ein Reinigungstensid, das anionisches Tensid und Betaine umfasst,
b) zu mindestens 0,05 Gewichtsprozent ein kationisches Polymer;
i) wobei das kationische Polymer ein Molekulargewicht von 10.000 bis 10.000.000 aufweist; und
ii) wobei das kationische Polymer eine Ladungsdichte von 1,4 mÄq/g bis 7 mÄq/g aufweist,
c) zu mindestens 0,05 Gewichtsprozent ein Konditioniermittelmaterial mit einem volumengemittelten Durchmesser von weniger als 1,0 Mikrometer; und
d) zu mindestens 20,0 Gewichtsprozent einen wässrigen Träger.

2. Zusammensetzung nach Anspruch 1, wobei das kationische Polymer ein kationisches Polysaccharid ist.

3. Zusammensetzung nach Anspruch 2, wobei das kationische Polysaccharid ausgewählt ist aus der Gruppe bestehend aus kationischen Anhydroglucosen, kationischen Guargummiderivaten, kationischen Stärken und kationischen Cellulosen.

4. Zusammensetzung nach Anspruch 1, wobei das Konditioniermittelmaterial Silikon enthält.

5. Zusammensetzung nach Anspruch 4, wobei das Silikon-Konditioniermittelmaterial eine Organosiloxan-Nanoemulsion ist.

6. Zusammensetzung nach Anspruch 5, wobei die Organosiloxan-Nanoemulsion einen volumengemittelten Durchmesser von weniger als 0,1 Mikrometer aufweist.

7. Zusammensetzung nach Anspruch 1, wobei das Konditioniermittelmaterial einen volumengemittelten Durchmesser von weniger als 0,3 Mikrometer, vorzugsweise weniger als 0,1 Mikrometer, mehr bevorzugt von weniger als 0,05 Mikrometer aufweist.

8. Zusammensetzung nach Anspruch 1, wobei das kationische Polymer ein Molekulargewicht von 50.000 bis 5.000.000, vorzugsweise von 100.000 bis 3.000.000 aufweist.

9. Zusammensetzung nach Anspruch 1, wobei das kationische Polymer eine Ladungsdichte von mindestens 1,6 mÄq/g, vorzugsweise mindestens 1,8 mÄq/g, am meisten bevorzugt mindestens 2,0 mÄq/g aufweist.

10. Zusammensetzung nach Anspruch 1, wobei das kationische Polymer eine Ladungsdichte von weniger als 4 mÄq/g aufweist.

11. Zusammensetzung nach Anspruch 1, wobei das Gewichtsverhältnis von dem kationischen Polymer zu dem Konditioniermittel von 4:1 bis 1:40, vorzugsweise von 2:1 bis 1:20, mehr bevorzugt von 1:1 1 bis 1:5 beträgt.

12. Zusammensetzung nach Anspruch 1, umfassend von 0,075 Gewichtsprozent bis 2 Gewichtsprozent das kationische Polymer, vorzugsweise von 0,1 Gewichtsprozent bis 1 Gewichtsprozent das kationische Polymer.

13. Zusammensetzung nach Anspruch 1, umfassend von 0,1 Gewichtsprozent bis 5 Gewichtsprozent, vorzugsweise von 0,2 Gewichtsprozent bis 3 Gewichtsprozent das Konditioniermittel.

14. Kosmetisches Verfahren zum Behandeln von Haar durch Verabreichung einer sicheren und wirksamen Menge der Zusammensetzung nach Anspruch 1.

## Revendications

1. Composition de shampooing comprenant :
a) de 4 à 50 pour cent en poids d'un agent tensioactif détersif comprenant un agent tensioactif anionique et des bétaïnes,
b) au moins 0,05 pour cent en poids d'un polymère cationique ;
i) dans laquelle ledit polymère cationique a une masse moléculaire allant de 10 000 à 10 000 000 ; et
ii) dans laquelle ledit polymère cationique a une densité de charge allant de 1,4 méq/g à 7 méq/g,
c) au moins 0,05 pour cent en poids d'un matériau de type agent de conditionnement ayant un diamètre moyen de volume de moins de 1,0 micron ; et
d) au moins 20,0 pour cent en poids d'un véhicule aqueux.

2. Composition selon la revendication 1, dans laquelle ledit polymère cationique est un polysaccharide cationique.

3. Composition selon la revendication 2, dans laquelle ledit polysaccharide cationique est choisi dans le groupe constitué d'anhydroglucoses cationiques, dérivés de gomme guar cationique, amidons cationiques, et celluloses cationiques.

4. Composition selon la revendication 1, dans laquelle ledit matériau de type agent de conditionnement contient de la silicone.

5. Composition selon la revendication 4, dans laquelle le matériau de type agent de conditionnement siliconé est une nanoémulsion d'organosiloxane.

6. Composition selon la revendication 5, dans laquelle ladite nanoémulsion d'organosiloxane a un diamètre moyen de volume de moins de 0,1 micron.

7. Composition selon la revendication 1, dans laquelle ledit matériau de type agent de conditionnement a un diamètre moyen de volume de moins de 0,3 micron, de préférence moins de 0,1 micron, plus préférablement de moins de 0,05 micron.

8. Composition selon la revendication 1, dans laquelle ledit polymère cationique a une masse moléculaire allant de 50 000 à 5 000 000, de préférence de 100 000 à 3 000 000.

9. Composition selon la revendication 1, dans laquelle ledit polymère cationique a une densité de charge d'au moins 1,6 méq/g, de préférence au moins 1,8 méq/g, le plus préférablement au moins 2,0 méq/g.

10. Composition selon la revendication 1, dans laquelle ledit polymère cationique a une densité de charge de moins de 4 méq/g.

11. Composition selon la revendication 1, dans laquelle le rapport pondéral dudit polymère cationique sur ledit agent de conditionnement va de 4:1 à 1:40, de préférence de 2:1 à 1:20, plus préférablement de 1:1 à 1:5.

12. Composition selon la revendication 1, comprenant de 0,075 pour cent en poids à 2 pour cent en poids dudit polymère cationique, de préférence de 0,1 pour cent en poids à 1 pour cent en poids dudit polymère cationique.

13. Composition selon la revendication 1, comprenant de 0,1 pour cent en poids à 5 pour cent en poids, de préférence de 0,2 pour cent en poids à 3 pour cent en poids dudit agent de conditionnement.

14. Procédé cosmétique de traitement des cheveux en administrant une quantité sûre et efficace de la composition selon la revendication 1.
